# DEMANDE DE BREVET EUROPEEN

(11) **EP 0 617 934 A1**
(43) Date de publication de la demande: **05.10.1994**
(21) Numéro de dépôt: 94400671.7
(22) Date de dépôt: 29.03.1994
(51) Int. Cl.: A61F 2/40

(54) **Tête pour prothèse modulaire d'épaule**

(30) Priorité: 30.03.1993 FR 9303824
(71) Demandeur: LANDANGER-LANDOS, Société Anonyme, F-52003 Chaumont (FR)
(72) Inventeur: Aubertot, Laurent, F-52200 Langres (FR); Kummar, Frederick J., NY - NY 10003 (US); Toggwiler, Hervé, F-89390 Aisy-sur-Armancon (FR); Zuckerman, Joseph D., NY - NY 10003 (US)
(74) Mandataire: Cabinet Martinet & Lapoux

(57) **Abrégé**

Une tête (233) pour prothèse modulaire d'épaule présente une surface extérieure sensiblement sphérique (237), et un téton (241) coopérant avec un trou le long d'un axe longitudinal (243) qui est incliné d'un angle prédéterminé par rapport à un axe longitudinal d'une tige dudit dispositif. La surface extérieure sensiblement sphérique (237) présente un centre de sphère (2C) qui est décentré par rapport à l'axe longitudinal (243) du téton (241). La surface sphérique s'étend dans un dièdre (180°-β) d'angle compris entre 150° et 170°. L'une des faces (239) du dièdre est sensiblement perpendiculaire à l'axe longitudinale (243).

## Description

La présente invention concerne un dispositif prothétique modulaire pour constituer une prothèse d'épaule.

Une prothèse partielle d'épaule, connue dans la technique antérieure, inclut de nombreuses configurations des différents composants comprenant la tête, le cou, le col, les mécanismes de blocage et la tige ou axe. Des prothèses visant le remplacement total de l'épaule sont également connues et comportent essentiellement un composant huméral qui est implanté dans l'humérus proximal et une cupule ou élément d'articulation implanté dans la cavité glénoïde de l'omoplate.

De nombreux types différents d'articulations d'épaule artificielle ont été proposés jusqu'ici.

D'un point de vue général, l'articulation d'épaule est relativement peu contrainte par rapport à la hanche. Elle inclut une tête et une glène assorties, mais la tête et la glène sont tenues dans leur position relative par un parement rotateur qui comprend une couche épaisse de muscles et de ligaments qui entoure l'articulation et qui contrôle également le mouvement général du bras par rapport au corps. Bien que l'épaule soit considérée comme une articulation ne supportant pas d'effort, la force compressive agissant sur l'articulation de l'épaule atteint presque le poids total du corps quand le bras est levé horizontalement, ce qui est désigné médicalement sous le nom d'abduction à 90 degrés. En conséquence, en entreprenant des tâches pesantes ou des activités athlétiques, l'épaule supporte fréquemment des charges sensiblement plus importantes que le poids du corps humain. L'épaule est donc une articulation majeure porteuse de charge.

Concernant la terminologie, l'os supérieur du bras est l'humérus, et la tête ou l'élément d'articulation arrondi, à l'extrémité supérieure de l'humérus, s'ajuste dans une glène de l'os de l'épaule ou omoplate. Le terme "glénoïde" se rapporte à la partie de l'omoplate qui reçoit la tête humérale. L'articulation d'épaule est donc parfois désignée sous le nom d'articulation gléno-humérale.

Des composants qui s'emboîtent et sont combinés dans un ensemble ont aussi été utilisés dans des prothèses d'épaule à la fois comme composant glénoïde et comme composant huméral. Un tel dispositif est connu sous le nom de prothèse modulaire. De plus, la technique antérieure enseigne l'utilisation d'une prothèse d'épaule partielle dans laquelle le composant huméral est d'une conception modulaire qui permet à des têtes de tailles appropriées différentes d'être assemblées de manière amovible à un élément de tige humérale qui a été implanté dans l'humérus proximal. La liaison détachable utilise un mécanisme de couplage entre la tête et la tige, qui peut comprendre un cône Morse. Cela permet à la tête humérale d'être insérée dans la tige par une partie de cou, qui s'ajuste dans la tige comme décrit dans la demande de brevet EP-A-0329854. Alternativement, la tige peut être conçue pour être insérée dans un orifice de la tête, ou un élément intermédiaire peut être biconique pour être introduit simultanément par ses deux côtés dans la tête et dans la tige.

Un article intitulé "The Geometry Of The Humeral Head And The Design Of Prosthesis" par Roberts et al. dans "The Journal Of Bone And Joint Surgery" de juillet 1991 donne une analyse dimensionnelle d'environ 30 humérus de cadavres.

Selon le résumé, la surface articulaire de la tête humérale est généralement décrite comme se présentant postéro-médialement, formant un angle entre environ 16 et 35 degrés avec le plan transépicondylien. En hémi-arthroplastie, la surface articulaire apparaît également comme décalée postérieurement par rapport à l'axe huméral. Une affection coracoïdienne peut apparaître si ce décalage n'est pas respecté. Une analyse a été faite sur 29 humérus de cadavre utilisant une machine de mesure de coordonnée industrielle. La position du centre de la tête a été définie par rapport à l'axe huméral et au plan transépicondylien. La surface articulaire humérale a été trouvée comme étant rétroversée de 21,4 degrés et son centre décalé postérieurement de 4,7 millimètres.

Des interprétations précédentes de la rétroversion n'ont pas tenu compte du déplacement vers le postérieur et cela peut être important dans la conception de future prothèse améliorée.

La présente invention vise à fournir une tête améliorée pour dispositif prothétique modulaire humérale satisfaisant les besoins anatomiques particuliers du patient beaucoup mieux que selon la technique antérieure.

A cette fin, une tête pour dispositif prothétique modulaire présente une surface extérieure sensiblement sphérique, et un moyen de montage de tête coopérant avec un moyen de montage de tige le long d'un axe longitudinal qui est incliné d'un angle prédéterminé par rapport à un axe longitudinal d'une tige dudit dispositif. La surface extérieure sensiblement sphérique présente un centre de sphère qui est décentré par rapport à l'axe longitudinal du moyen de montage de tête. La tête est caractérisée en ce que la surface extérieure sensiblement sphérique s'étend sensiblement dans un dièdre dont l'angle est compris entre 150° et 170°, de préférence 160°, et dont l'une des faces est sensiblement perpendiculaire à l'axe longitudinal du moyen de montage de tête.

Le dièdre a de préférence une arête qui est sensiblement concourante avec l'axe longitudinal du moyen de montage de tête.

La surface extérieure sensiblement sphérique peut avoir un rayon qui passe par le centre d'une base du moyen de montage de tête et forme un angle avec l'axe longitudinal du moyen de montage de tête compris entre 10° et 30°.

Avantageusement, la tête comporte un bord intérieur qui a une surface extérieure lisse arrondie ou plane qui s'étend entre la surface extérieure sensiblement sphérique et une surface plane de la tête sensiblement perpendiculaire à l'axe longitudinal du moyen de montage de tête.

De préférence, le rayon de la surface extérieure sensiblement sphérique est d'une longueur comprise entre environ 20 et 30 millimètres, de préférence 25 millimètres.

Selon un autre aspect de l'invention, le décentrage du centre de la surface sphérique par rapport à l'axe longitudinal du moyen de montage est compris entre 2,5 et 7,5 millimètres environ, de préférence 5 millimètres.

Le moyen de montage de tête peut être un téton ou un trou tronconique, du type cône Morse de blocage afin d'assembler facilement et rapidement la tête à la tige du dispositif.

D'autres avantages et caractéristiques de l'invention apparaîtront plus clairement à la lecture de plusieurs réalisations préférées de l'invention en référence aux dessins annexés, dans lesquels :
- la figure 1 est une vue de face latérale de la partie de tige du composant huméral selon la présente invention ;
- la figure 2 est une vue de côté antérieur ou postérieur dans un plan médio-latéral, de la partie de tige du composant huméral selon la présente invention ;
- la figure 3 est une vue de dessus de la partie de tige prise le long de la ligne III-III de la figure 2 ;
- la figure 4 est une section selon la ligne IV-IV de la figure 1 ;
- la figure 5 est une vue de côté d'une première tête selon la technique antérieure d'un composant huméral ;
- la figure 6 est une vue de côté d'une seconde tête selon la technique antérieure d'un composant huméral ;
- la figure 7 est une vue de côté de la tête du composant huméral selon une première version de première réalisation de la présente invention ;
- la figure 8 est une vue de côté de la tête du composant huméral selon une seconde version de première réalisation de la présente invention ;
- la figure 9 est une vue de côté de la tête du composant huméral selon une première version de seconde réalisation de la présente invention ; et
- la figure 10 est une vue de côté de la tête du composant huméral selon une seconde version de seconde réalisation de la présente invention.

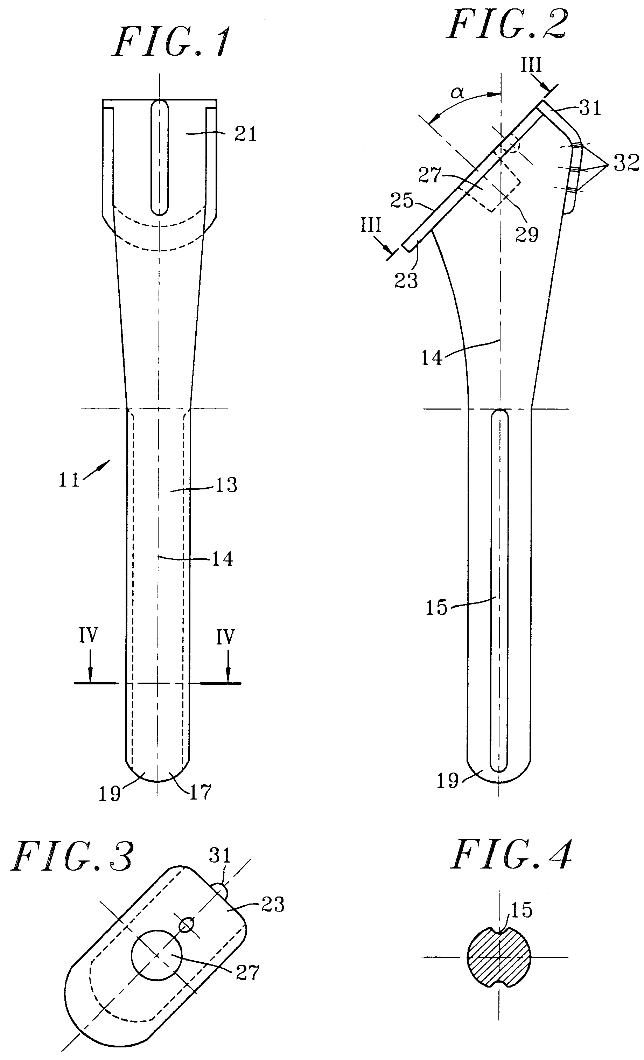

En référence aux figures 1 à 4, un composant huméral modulaire selon la présente invention est sous la forme d'une tige humérale 11 ayant une partie longiligne 13. La partie longiligne 13 a un axe longitudinal 14 et des rainures opposées, longilignes 15 de chaque côté de la partie longiligne par rapport à un plan de symétrie médio-latéral. Les rainures 15 contribuent à la fixation de la tige 11 dans l'humérus et procurent une stabilité contre toute rotation de la prothèse.

L'extrémité distale 17 de la tige 11 a une partie arrondie 19, et l'extrémité proximale 21 de la tige 11 a une face d'appui 23 qui s'étend transversalement à la tige 11. La face d'appui 23 a une surface plane supérieure 25, et un trou 27 s'étendant vers l'intérieur depuis la surface plane supérieure 25. Le trou 27 constitue un moyen de montage de tige et a un axe longitudinal 29 qui est incliné par rapport à l'axe longitudinal 14 de la partie longiligne 13 de la tige 11. L'inclinaison est définie par un angle prédéterminé "α" qui est de 45 degrés dans la réalisation préférée. De même, la surface plane supérieure 25 de la face d'appui 23 est inclinée d'un angle prédéterminé, complémentaire de l'angle "α", par rapport au même axe longitudinal 14, l'angle complémentaire étant de préférence de 45 degrés. L'axe de trou 29 est ainsi perpendiculaire à la surface 25 de la face d'appui 23.

La tige 11 inclut également un bourrelet latéral 31 ayant des évidements de fixation 32 qui servent à la fixation du composant huméral après avoir été introduit dans l'humérus.

Selon une variante de réalisation non représentée, la tige 11 comprend, sur une partie de sa longueur, une série de collerettes formant entr'elles des gorges, régulièrement réparties longitudinalement.

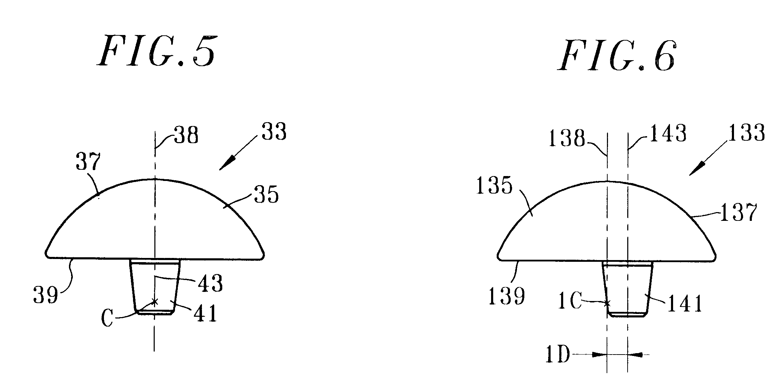

En référence maintenant à la figure 5, une première tête conventionnelle 33 pour dispositif modulaire prothétique comprend une calotte sphérique 35 et un téton de centrage 41. La calotte 35 présente une surface extérieure de forme sphérique 37, ayant un axe central de symétrie 38, et une surface plane inférieure 39. Un téton de centrage conique constituant un moyen de blocage et montage de tête 41, complémentaire du trou 27, saille vers le bas depuis le centre de la surface plane inférieure 39 de la tête 33. Le téton de montage de tête est de préférence formé en une seule pièce avec la calotte 35, et est disposé de telle sorte que l'axe longitudinal 43 du téton 41 soit confondu avec l'axe central 38 de la surface 35. En d'autres termes, le centre C de la surface sphérique 37 est centré sur l'axe de téton 43.

Le montage de la tête sur la tige 11 est obtenu par emmanchement à force du téton 41 dans le trou 27. Le téton 41 et le trou 27 sont de préférence sous forme de cônes Morse de blocage conventionnels respectivement mâle et femelle, qui produisent un assemblage par friction quand ils sont connectés ensemble. Après cette connexion, la surface plane inférieure 39 de la tête 33 est en butée contre la surface plane supérieure 25 de la tige 11, et l'axe longitudinal 43 du téton de blocage et montage 41 coïncide avec l'axe 29 du trou 27. Par conséquent, d'une manière analogue aux inclinaisons de l'axe 29 du trou 27 et de la surface d'appui 23 par rapport à l'axe longitudinal de tige 14, l'axe 43 du téton 41 est incliné par rapport à l'axe longitudinal 14 de la partie longiligne 13 de la tige 11 d'un angle prédéterminé "α", qui est de 45 degrés comme indiqué précédemment.

La surface extérieure de forme sphérique 37 de la tête 33 est adaptée pour s'engager dans la cavité glénoïde elle-même ou dans une prothèse équivalente. Le centre de la surface sphérique 37 se trouve le long de son axe central de symétrie 38.

En référence maintenant à la figure 6, une seconde tête conventionnelle 133 présente une calotte 135 ayant une surface extérieure de forme sphérique 137, un axe central de symétrie 138 de la surface extérieure de forme sphérique 135, et une surface plane inférieure 139. Un moyen de blocage de montage 141, sous la forme également d'un téton tronconique, saille vers le bas depuis la surface plane inférieure 139 de la tête 133 et est de préférence formée en une seule pièce avec la tête 133. Le téton de blocage conique 141 est disposé de telle sorte que l'axe longitudinal 143 de celui-ci est parallèle et séparé par un entraxe 1D depuis l'axe central 138 de la calotte 135 et donc depuis l'axe longitudinal 43 du téton de blocage de montage 41 représenté à la figure 5, lorsque les calottes 35 et 135 sont identiques. Dans cette réalisation, le centre 1C de la surface sphérique 137 est ainsi décentré par rapport à l'axe longitudinal 143 du téton de montage 141.

Le montage du dispositif selon la réalisation de la figure 6 est réalisé par emmanchement à force du téton de tête 141 dans le trou de tige 27. Le téton 141 et le trou 27 sont de préférence sous la forme de cônes Morse de blocage conventionnels respectivement mâle et femelle qui produisent un assemblage par friction lorsqu'ils sont connectés ensemble. Après cet assemblage, la surface plane inférieure 139 de la tête 133 est en butée contre la surface plane supérieure 25 de la tige 11, les surfaces 25 et 139 étant superposées perpendiculairement aux axes confondus 29 et 143.

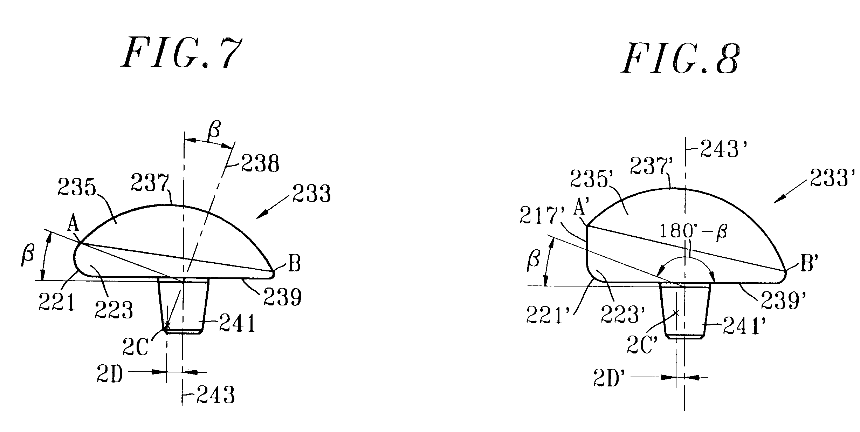

En référence à la première version de la première réalisation montrée à la figure 7, une tête prothétique 233 de la présente invention comprend une calotte sensiblement sphérique 235 et un téton de montage 241. La calotte 235 a une surface supérieure sphérique 237 qui surmonte un coin sensiblement à contour cylindrique arrondi dont une face supporte la surface 237 et dont une autre face plane 239 est destinée à buter contre la surface d'appui de tige 25. Ainsi, la surface extérieure sphérique 237 s'étend depuis un point A à l'extrémité supérieure de la surface arrondie lisse 221 d'un bord intérieur (médian) 223 du coin vers un point B au bord extérieur (latéral) du coin quasiment confondu avec celui de la surface 239.

Le téton de montage 241 saille vers le bas depuis et est perpendiculaire à la surface plane inférieure 239 de la tête 233 et est de préférence formé en une seule pièce avec la tête 233. Le téton de montage tronconique 241 est disposé de telle sorte que son axe longitudinal 243 passe approximativement par le centre de la surface plane inférieure 239. Le centre 2C de la surface sphérique 237 est décentré d'une distance 2D par rapport à l'axe longitudinal 243 du téton de montage 241. La distance 2D est comprise entre 2,5 et 7,5 millimètres environ et vaut de préférence environ 5 millimètres. Selon la figure 7, le centre 2C est situé vers "l'intérieur", c'est-à-dire à gauche de l'axe de téton de montage 243.

Dans la figure 7, il apparaît que la surface sphérique 237 de calotte est comprise sensiblement dans un dièdre dont l'arête concourt avec l'axe longitudinal de téton 243, sensiblement au centre de la grande base du téton 243, dont l'un des demi-plans ou faces est défini par une partie de la surface inférieure 239, à droite dans la figure 7, et dont l'angle 180°-β est compris entre 160° et 170° environ. Selon la réalisation illustrée, un rayon 238 de la surface sphérique 237 issu du centre 2C passe par le centre de la grande base du téton 241 et forme un angle d'environ β avec l'axe de téton 243.

L'angle "β" est de préférence d'environ 20 degrés mais peut être compris entre 10 et 30 degrés. Dans cette réalisation, le rayon de la surface extérieure de forme sphérique 237 est compris entre 20 et 30 millimètres et est typiquement égal à 25 millimètres.

Pour assembler la tige 11 avec la tête 233 selon la version de la figure 7, le téton de montage 241 est emmanché à force dans le trou de tige 27. Le téton 241 et le trou 27 sont de préférence sous la forme de cônes Morse de blocage conventionnels respectivement mâle et femelle qui produisent un assemblage par friction lorsqu'ils sont connectés ensemble. Après cet assemblage, la surface plane inférieure 239 de la tête 233 est en butée contre la surface plane supérieure 25 de la tige 11.

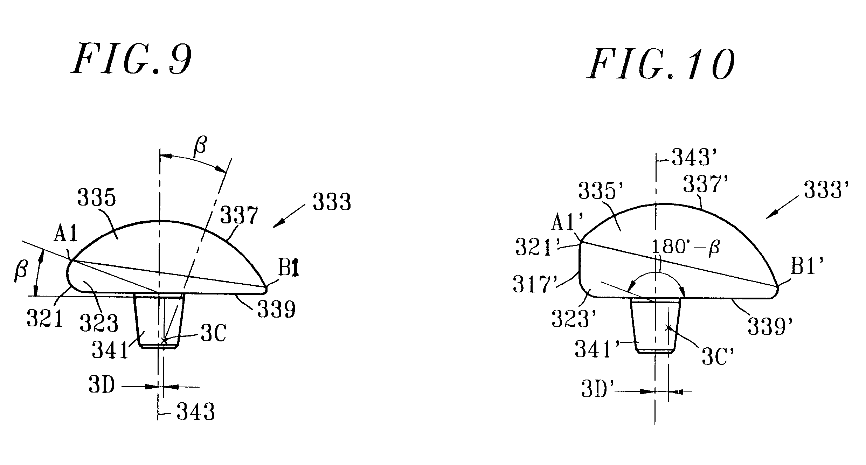

En référence maintenant à la figure 8, dans laquelle une seconde version de la première réalisation de la tête 233' est représentée, il est à noter que son profil est sensiblement le même que celui de la première version montrée dans la figure 7 et en conséquence seulement quelques-unes de ses différences sont décrites. La tête 233' a une surface extérieure sensiblement cylindrique 221' sur le bord intérieur 223' d'un coin inférieur de la tête 233' et une surface extérieure 237' de calotte 235' de forme sphérique. La surface 237' s'étend depuis un point A' à l'extrémité supérieure du bord intérieur 223' vers un point B' juste au-dessus du bord extérieur de la surface plane inférieure 239'. La surface extérieure 221' inclut une partie relativement longue 217' qui est située entre des parties courbes courtes supérieure et inférieure et qui offre un profil rectiligne parallèle à l'axe 243' du téton 241'. La surface sphérique est encore contenue dans un dièdre d'angle 180°-β comme défini précédemment.

En référence maintenant à la figure 9, une seconde réalisation de la tête 333 de la présente invention est représentée. La tête 333 est composée, de préférence d'une manière monolithique, d'une calotte sphérique 335, d'un téton de montage tronconique 341, et d'un coin sensiblement cylindrique situé entre la calotte 335 et le téton 341. La surface extérieure lisse 321 du coin est arrondie. La surface extérieure sphérique 337 de la calotte 335 s'étend depuis un point A1 à l'extrémité supérieure du bord intérieur de coin 323 vers un point B1 au bord extérieur du coin et d'une surface plane inférieure de tête 339. Le téton de montage 341 saille vers le bas depuis la surface plane inférieure 339 de la tête 333, et est disposé de telle sorte que son axe longitudinal 343 soit séparé par un entraxe 3D, de préférence approximativement 5 millimètres, depuis le centre 3C de la surface sphérique 337 et du centre de la surface inférieure 339. Comparativement à la figure 7, le centre de surface sphérique est situé vers l'extérieur (latéral), c'est-à-dire à droite, par rapport à l'axe de téton 343. L'entraxe 3D peut être compris entre 2,5 à 7,5 millimètres.

Comme dans la figure 7, un dièdre d'angle 180°-β contient la surface sphérique 337 et est défini par une face confondue avec la partie de droite de la surface inférieure 339, et par une arête passant sensiblement par l'axe longitudinal 343 du téton 341 et le centre de la grande base du téton 341. L'angle "β" est de préférence d'environ 20 degrés mais peut être compris entre 10 et 30 degrés. Dans cette réalisation, le rayon de la surface extérieure de forme sphérique 335 est de préférence de 25 millimètres.

Le montage, selon la version montrée à la figure 9, est encore obtenu par un emmanchement à force du téton 341 dans le trou 27. Le téton 341 et le trou 27 sont de préférence sous la forme de cônes Morse de blocage conventionnels respectivement mâle et femelle qui produisent un assemblage par friction lorsqu'ils sont connectés ensemble. Après connexion, la surface plane inférieure 339 de la tête 333 est en butée contre la surface plane supérieure 25 de la tige 11.

En référence maintenant à la figure 10, dans laquelle une seconde version de la seconde réalisation de la tête 333' est représentée, on note que son profil est sensiblement analogue à celui de la première version de la seconde réalisation montrée dans la figure 9 et en conséquence seulement quelques-unes de ses différences sont décrites. La tête 333' a une surface sensiblement cylindrique 321' sur son bord intérieur 323' d'un coin inférieur intermédiaire entre une calotte 335' et un téton 341'. La calotte 335' a une surface extérieure de forme sphérique 337' qui est contenue dans un dièdre d'angle 180°-β et s'étend depuis le point A1' à l'extrémité supérieure du bord intérieur 323' vers le point B1' juste au-dessus du bord extérieur de la surface plane inférieure 339'. La surface extérieure 321' inclut une partie relativement longue 317' qui est située entre des parties courbes courtes supérieure et inférieure et qui offre un profil rectiligne parallèle à l'axe 343' du téton 341'.

Selon une variante de réalisation non représentée, le dispositif comprend, au lieu d'un téton 241, 341, un trou dans la tête, et au lieu d'un trou 27, un téton de montage conique saillant de la surface 25 de la tige et complémentaire du trou de tête.

Selon encore une autre variante, le moyen de montage, saillant respectivement soit sous la tête, soit sur la tige, est cylindrique et comprend un petit anneau souple ou fendu cerclant une petite gorge pour pénétrer à force dans une gorge du trou complémentaire du moyen de montage, formé respectivement soit sur la tige, soit sur la tête.

Dans tous les cas, le chirurgien, après implantation de la tige 11 dans l'humérus, choisit l'une des têtes 233, 233', 333 ou 333', et ajuste la position de celle-ci par rotation par rapport à la tige, pour adapter la prothèse à l'anatomie du patient. Une fois la position relative de la tête et de la tige déterminée, le téton de montage est emmanché à force dans le trou 27, et ainsi la tête et la tige sont liées rigidement en rotation et en translation.

## Revendications

**1 -** Tête (133, 233, 333) pour dispositif modulaire prothétique,
ladite tête présentant une surface extérieure sensiblement sphérique (137, 237, 337), et un moyen de montage de tête (141, 241, 341) coopérant avec un moyen de montage de tige (27) le long d'un axe longitudinal (143, 243, 343 ; 29) qui est incliné d'un angle prédéterminé (α) par rapport à un axe longitudinal (14) d'une tige (11) dudit dispositif,
la surface extérieure sensiblement sphérique (137, 237, 337) présentant un centre de sphère (1C, 2C, 3C) qui est décentré par rapport à l'axe longitudinal (143, 243, 343) du moyen de montage de tête (141, 241, 341),
caractérisée en ce que la surface extérieure sensiblement sphérique (237, 337) s'étend sensiblement dans un dièdre dont l'angle (180°-β) est compris entre 150° et 170°, de préférence 160°, et dont l'une (239, 339) des faces est sensiblement perpendiculaire à l'axe longitudinal (243, 343) du moyen de montage de tête (241, 341).

**2 -** Tête conforme à la revendication 1, dans laquelle le dièdre a une arête qui est sensiblement concourante avec l'axe longitudinal (243, 343) du moyen de montage de tête (241, 341).

**3 -** Tête conforme à la revendication 1 ou 2, dans laquelle la surface extérieure sensiblement sphérique (237) a un rayon (238) qui passe par le centre d'une base du moyen de montage de tête (241), et forme un angle (β) avec l'axe longitudinal (243) du moyen de montage de tête (241) compris entre 10° et 30°.

**4 -** Tête conforme à l'une quelconque des revendications 1 à 3, comportant un bord intérieur (223, 323) qui a une surface extérieure lisse arrondie (221, 321) ou plane (221', 321') qui s'étend entre la surface extérieure sensiblement sphérique (235, 335) et une surface plane (239, 339) de la tête sensiblement perpendiculaire à l'axe longitudinal (243, 343) du moyen de montage de tête (241, 341).

**5 -** Tête conforme à l'une quelconque des revendications 1 à 4, dans lequel le rayon de ladite surface extérieure sensiblement sphérique (237, 337) est d'une longueur comprise entre environ 20 et 30 millimètres, de préférence 25 millimètres.

**6 -** Tête conforme à l'une quelconque des revendications 1 à 5, dans laquelle le décentrage (2D, 3D) du centre de la surface sphérique par rapport à l'axe longitudinal (243, 343) du moyen de montage est compris entre 2,5 et 7,5 millimètres environ, de préférence 5 millimètres.

**7 -** Tête conforme à l'une quelconque des revendications 1 à 6, dans laquelle ledit moyen de montage de tête (241, 341) est conique.

**8 -** Tête conforme à l'une quelconque des revendications 1 à 6, dans laquelle ledit moyen de montage de tête comprend un cône Morse de blocage (241, 341).

**9 -** Tête conforme à l'une quelconque des revendications 1 à 6, dans laquelle ledit moyen de montage de tête comprend un trou tronconique.
